# EUROPEAN PATENT SPECIFICATION

(11) **EP 2 877 149 B1**
(45) Date of publication and mention of the grant of the patent: **09.08.2017**
(21) Application number: 13758978.4
(22) Date of filing: 18.07.2013
(51) Int. Cl.: A61K 8/35, A61K 8/365, A61K 8/46, A61Q 5/04, A61Q 5/06

(54) **SEMI-PERMANENT MODIFICATION OF THE STRUCTURE OF THE KERATIN FIBRES OF THE HAIR THROUGH THE COMBINED USE OF HEAT AND MIXTURES OF ORGANIC SUBSTANCES**
SEMIPERMANENTE MODIFIKATION DER STRUKTUR DER KERATINFASERN DES HAARES DURCH DEN KOMBINIERTEN EINSATZ VON WÄRME UND MISCHUNGEN AUS ORGANISCHEN STOFFEN
MODIFICATION SEMI-PERMANENTE DE LA STRUCTURE DES FIBRES DE KÉRATINE DES CHEVEUX PAR LE BIAIS DE L'UTILISATION COMBINÉE DE CHALEUR ET DE MÉLANGES DE SUBSTANCES ORGANIQUES

(30) Priority: 25.07.2012 IT AP20120007
(43) Date of publication of application: 03.06.2015
(73) Proprietor: D'Ottavi, Adele, 63048 Montemonaco (AP) (IT)
(72) Inventor: D'Ottavi, Adele, 63048 Montemonaco (AP) (IT)
(74) Representative: Statti, Francesco
(86) International application number: PCT/IB2013/001566
(87) International publication number: WO 2014/016658

(56) References cited:
- WO-A1-2010/141098
- WO-A2-2011/104282
- KR-A- 20100 029 567
- US-A1- 2012 031 420

## Description

### Field of the Invention

New formulations suitable for semi-permanently modifying hair and keratin fibres in general through the combined use of several organic substances and heat are the subject of the present patent application.

In particular, the organic substances used are the following: dihydroxyacetic acid, monohydroxyacetone and methylsulfonylmethane, while as regards the heat, the temperatures are between 170°C and 230°C.

### Background of the Invention

The structure of human hair is well described in US 6517822 B1. Similar structures are animal hairs, such as wool for example.

In the treatment of frizzy or curly hair to straighten it, numerous chemical substances that can interact with the amino acids making up the hair fibre are used. These treatments are well described, for example, in WO 2007/135299 A1 and in US 2010/0300471 A1.

WO 2007/135299 A1 and FR 2901472 A1 describe a method for straightening keratin fibres using high temperature and particular families of carboxylic acids (alpha-hydroxylic and alpha-ketonic) and their derivatives. WO 2010/141098 A1 describes a formulation for conditioning hair containing various chemical substances, also including methylsulfonylmethane (CAS 67-71-0).

KR20100013791 A describes the use of formulations containing amino acids, proteins and other organic substances including hydroxyacetone to strengthen and modify the keratin fibres.

In WO2011/104282 A2 and in WO2012/010351 A2, examples are given of the application of formulations containing glyoxylic acid in combination with other organic substances that produce a semi-permanent conditioning of hair fibres lasting for several washes with water and shampoo.

SU537115 A1 describes the use of formulations containing glyoxylic acid to change the shape of animal hairs (fur).

WO 2011/104282 A2 describes a process that consists of applying aqueous solutions of formulations containing glyoxylic acid, alternately in combination with potassium iodide, dihydroxyacetone (CAS 96-26-4), ozonized jojoba oil and glycerol, 2,4 hexadienal and subsequent heating with hair iron heated to a temperature of about 200 +/-50 °C.

WO 2011/104282 A2 differs from the present patent application since it does not envisage the use of a formulation containing dihydroxyacetic acid, methylsulfonylmethane and monohydroxyacetone, combined in aqueous solution at a concentration of between 0.1 % and 25% by weight, to achieve the technical advantages highlighted by the research activity performed. WO 2011/104282 A2 envisages the use of formulations containing dihydroxyacetone (CAS 96-26-4) in combination with glyoxylic acid. The present patent application discloses formulations that always contain in combination the three components dihydroxyacetic acid (CAS 563-96-2), monohydroxyacetone (CAS 116-09-6) and methylsulfonylmethane (CAS 67-71-0), therefore differing from WO 2011/104282 A2.

US 2012/0031420 describes a process that consists of applying formulations containing concentrations of mercaptosilicone homopolymer or mercaptosilicone copolymer of between 0.1% and 20% by weight, resulting from the effect of high temperatures on the mercaptosilicone monomer.

US 2012/0031420 A1 envisages the use of a variable concentration of between 1% and 10% of methylsulfonylmethane as an alternative to variable concentrations of between 1% and 10% of urea (paragraph 0010). The optional presence of methylsulfonylmethane in the formulation and the function that this molecule can have in the process are therefore not justified by scientific evidence, and the further beneficial effect it induces, other than those given by the class of substances on which the patent application is centred, is not specified.

WO 2010/141098 A1 describes a process that consists of applying a formulation that, among the various substances, also contains methylsulfonylmethane at concentrations of between 0.2% and 10% by weight and subsequent heat straightening with heated hair iron. In the formulation that is the subject of WO 2010/141098 A1, neither dihydroxyacetic acid nor monohydroxyacetone are present. The benefits of using methylsulfonylmethane are combined with those of other chemical substances with the objective of obtaining a formulation that does not damage the hair even after repeated treatments and is not characterized by particularly high pHs with consequent irritation of the scalp. The benefits of using methylsulfonylmethane are however known in the prior art. KR 2010/0029567 A describes the use of enzymes (transglutaminases) and other substances that can chemically bind to the amino acids of the keratin chains of hair fibres, among which hydroxyacetone is also mentioned. In the composition of the formulations of KR 2010/0029567 A, neither dihydroxyacetic acid nor methylsulfonylmethane are present; furthermore, the patent does not specify anything with regard to how long the treatment lasts and demonstrates nothing of the presumed ability of hydroxyacetone to chemically bind to the amine residues of the amino acids present in keratin.

### Disclosure of the Invention

Surprisingly, it has been discovered that the combined use of Dihydroxyacetic acid, monohydroxyacetone and methylsulfonylmethane can potentiate the effect of the individual substances, thereby making curly and/or frizzy hair straight in a particularly efficient, effective and long-lasting way and lasting for at least ten washes with water and shampoo.

The results obtained using mixtures of the substances that are the subject of this patent application show that the separate use of the substances that are the subject of the formulations for which protection is being requested provide lower quality performance than that which can be seen with the invention that is the subject of this application.

With the combined application of the formulations described below and heat on human keratin fibres (curly and/or frizzy hair), straight, soft, shiny hair is obtained (and with considerable reduction in the volume of frizzy hair), which maintains these characteristics even after repeated washes with water and shampoo (semi-permanent effect).

The process according to the invention modifies the spatial conformation of the hair fibres, thereby reducing their occupied volume and the number of waves, through modification of the keratin structure.

In particular, analyses carried out by differential scanning calorimetry (DSC) showed a modification of the relationship between alpha and beta keratin. More precisely, the process in which the formulations described in the following examples have been used envisages the following steps:
- application on the hair fibres of a formulation containing dihydroxyacetic acid, monohydroxyacetone and methylsulfonylmethane, in addition to substances normally used in cosmetics (such as, for example, emulsifiers, pH buffers, emollients, solvents, perfume, etc.);
- leaving the substance in contact with the hair for between 15 and 60 minutes;
- drying the hair;
- straightening the hair using a hair straightening iron set to a temperature of about 200°C (+/- 30°C).

In particular, the process according to the invention has proved to be able to induce semi-permanent changes in the structure of the hair, which remain such even after repeated washes of the hair in normal conditions of usc.

### Detailed Description of the Invention

Further features of the invention will appear clearer from the detailed description that follows, related to an embodiment that is purely an example and therefore non-limiting.

Some examples of formulations are given below:

### Formulation example no. 1:

- Dihydroxyacetic acid 5 grams
- Methylsulfonylmethane 1 gram
- Monohydroxyacetone 1 gram
- Cetostearyl alcohol 0.5 grams
- Cetrimonium chloride 0.5 grams
- Perfume 0.5 grams
- Water 91.5 grams

### Formulation example no. 2:

- Dihydroxyacetic acid 10 grams
- Methylsulfonylmethane 2 grams
- Monohydroxyacetone 2 grams
- Cetostearyl alcohol 0.5 grams
- Cetrimonium chloride 0.5 grams
- Perfume 0.5 grams
- Water 84.5 grams

### Formulation example no. 3:

- Dihydroxyacetic acid 20 grams
- Methylsulfonylmethane 4 grams
- Monohydroxyacetone 4 grams
- Cetostearyl alcohol 1 gram
- Cetrimonium chloride 0.5 grams
- Perfume 1 gram
- Water 70.5 grams

Formulations containing substances known for their ability to interact with hair fibres (formulations 4 and 5) were also made.

### Formulation example no. 4:

- Glyoxylic acid 10 grams
- Cetostearyl alcohol 1 gram
- Cetrimonium chloride 0.5 grams
- Perfume 1 gram
- Water 87.5 grams

### Formulation example no. 5:

- Glyoxylic acid 10 grams
- Acetoin 1 gram
- Diacetyl 1 gram
- Cetostearyl alcohol 1 gram
- Cetrimonium chloride 0.5 grams
- Perfume 1 gram
- Water 85.5 grams

Formulations containing the individual substances making up the formulation that is the subject of the present patent application were also made.

### Formulation example no. 6:

- Monohydroxyacetone 2 grams
- Cetostearyl alcohol 1 gram
- Cetrimonium chloride 0.5 grams
- Perfume 1 gram
- Water 95.5 grams

### Formulation example no. 7:

- Methylsulfonylmethane 4 grams
- Cetostearyl alcohol 1 gram
- Cetrimonium chloride 0.5 grams
- Perfume 1 gram
- Water 93.5 grams

### Formulation example no. 8:

- Dihydroxyacetic acid 10 grams
- Cetostearyl alcohol 1 gram
- Cetrimonium chloride 0.5 grams
- Perfume 1 gram
- Water 87.5 grams

The formulations were applied with a stiff bristle brush to locks of hair about 25 cm long and weighing 3 grams each.

The hair fibres were left in contact with the above-mentioned solutions for 30 minutes, after which they were dried with a hair dryer in the normal way. Once dried, the hair fibres were straightened using a flat hair iron, normally used in cosmetics, heated to a temperature of about 210 degrees centigrade. At the end of the treatment with the eight different formulations, all the locks of hair fibres were washed with water and shampoo and dried with a hair dryer in the normal way.

After the first wash, all the locks of hair fibres were shiny, straight and soft to the touch.

They were then washed with water and shampoo and dried with a hair dryer in the normal way twelve times.

For formulations 1, 2 and 3, substantial maintenance of the straight shape was observed even after the twelfth wash, especially for formulations no. 2 and 3. The locks of hair treated with formulations no. 4 and 5 showed a gradual reduction in the straightening effect that tended to disappear after six or seven washes.

The locks of hair treated with formulations no. 6 and 7 showed a rapid reduction in the straightening effect with return to the original curly and frizzy appearance after only two or three washes with water and shampoo.

The locks of hair treated with formulation no. 8 showed a gradual reduction in the straightening effect that tended to disappear after seven or eight washes. Subsequent tests carried out with different times for contact of the formulations with the keratin fibres (15 and 60 minutes) showed a reduction in the straightening capacity of the respective formulations for contact times of 15 minutes and basically identical performance for contact times of 60 minutes compared to 30 minutes.

The results obtained show that a formulation containing dihydroxyacetic acid, monohydroxyacetone and methylsulfonylmethane can change the shape of human hair in a more effective and lasting way than other formulations already known for their power to interact with the keratin fibres of the hair.

### Definitions

Semi-permanent conditioning: treatment of curly or frizzy hair to make it straight, and with it staying in this condition for at least six washes with water and shampoo.

Dihydroxyacetic acid: organic substance having CAS number 563-96-2, empirical formula C₂H₃O₄, structural formula: 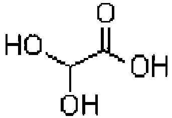 no INCI name given, and the following synonyms: glyoxylic acid monohydrate.

Dihydroxyacetic acid is a substance produced by the reaction between glyoxylic acid and water according to the following diagram:

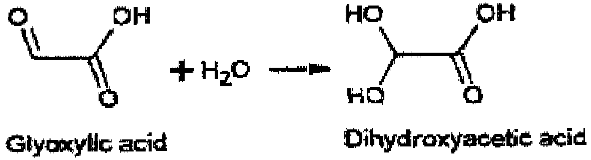

The reaction modifies the chemical structure of the glyoxylic acid by binding, with covalent bonds, a water molecule to the aldehyde group (-C=O) with its consequent transformation into two alcohol groups (-C-OH).

It is therefore not merely a hydration of glyoxylic acid, but a true chemical reaction that stably and permanently transforms glyoxylic acid (molecular weight 74) into dihydroxyacetic acid (molecular weight 91). Monohydroxyacetone: organic substance having CAS number 1.16-09-6, empirical formula C₃H₆O₂, structural formula: 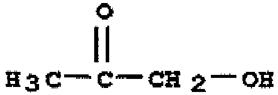 no INCI name given, and the following synonyms: 1-hydroxyacetone, acetol. Methylsulfonylmethane: organic substance having CAS number 67-71-0, empirical formula C₂H₆O₂S, structural formula:

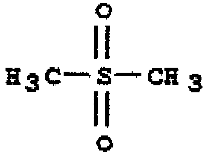

INCI name Dimethyl Sulfone, and the following synonyms: Methylsulfonylmethane, Sulfonyldimethane.

## Claims

1. Formulation for cosmetic use, suitable for semi-permanently straightening the keratin fibres making up human hair comprising dihydroxyacetic acid, monohydroxyacetone and methylsulfonylmethane.

2. Formulation according to claim 1 wherein each of the said substances is present in the formulation at a concentration of between 0.1% and 25%.

## Patentansprüche

1. Rezeptur zur kosmetischen Anwendung, geeignet zum semipermanenten Glätten der Keratinfasern, aus denen sich menschliche Haare zusammensetzen, bestehend aus Dihydroxyessigsäure, Monohydroxyaceton und Methylsulfonylmethan.

2. Rezeptur nach Anspruch 1, wobei jeder der genannten Wirkstoffe zu einer Konzentration von 0,1% bis 25% in der Rezeptur enthalten ist.

## Revendications

1. Formulation à usage cosmétique, conçue pour le lissage semi-permanent des fibres de kératine constituant les cheveux humains, comprenant de l'acide dihydroxyacétique, de la monohydroxyacétone et du méthylsulfonylméthane.

2. Formulation selon la revendication 1, dans laquelle chacune de ces substances est présente dans la formulation à une concentration se situant entre 0.1% et 25%.
